Europäisches Patentamt

(19) European Patent Office

Office européen des brevets

(11) Publication number: **0 276 939**
**A2**

(12) **EUROPEAN PATENT APPLICATION**

(21) Application number: **88300391.5**

(22) Date of filing: **19.01.88**

(51) Int. Cl.⁴: **A 61 M 16/16**
**A 61 M 15/00**

(30) Priority: **29.01.87 GB 8701957**

(43) Date of publication of application:
**03.08.88 Bulletin 88/31**

(84) Designated Contracting States:
**DE ES FR GB IT SE**

(71) Applicant: **LIFELINE LIMITED**
**Braye Road**
**Vale Guernsey Channel Islands (GB)**

(72) Inventor: **Bellm, Howard Geoffrey**
**The Priory**
**Binfield Berkshire (GB)**

(74) Representative: **Wright, Hugh Ronald et al**
**Brookes & Martin High Holborn House 52/54 High Holborn**
**London WC1V 6SE (GB)**

(54) **Nebulizer.**

㉗ A nebulizer for introducing a liquid in fine droplet form into a gas flow, including a reservoir for the liquid, a side wall, a base wall of generally conical shape, the base wall extending from a lower outer periphery adjacent said side wall to a central raised part, the base wall being covered by a flange plate whereby there is provided a capillary path from the lower outer periphery to a centrally mounted jet.

EP 0 276 939 A2

## Description

### NEBULIZER

The present invention relates to nebulizers. Nebulizers are used to introduce a liquid in fine droplet form into a gasilow, typically a liquid in the form of medication which is to be entrained in an air or oxygen gas flow and which is then breathed by a patient whereby the medication is applied to the patient through the lungs.

Nebulizers have become widely used in hospitals and other medical situations and in particular, are often used by elderly people in their homes. The nebulizer includes a reservoir for the liquid (generally water) and because of the dexterity required it is sometimes difiicult for an elderly person to fill the reservoir with the required accurate quantity of water or to take the nebulizer apart for cleaning. Furthermore nebulizers tend to be quite long, and in addition problems have been found whereby the connection of an oxygen tube to the nebulizer and movements of the patient have caused the jet arrangement within the nebulizer to become misaligned and not function properly. The usual nebulizer also includes a cap which is screwed onto the base part. The use of screw threads is inconvenient, particularly for less dextrous elderly people.

It would, therefore, be desirable to provide a compact more useful design of nebulizer which mitigates or overcomes one or more of the above problems.

The present invention provides a nebulizer comprising a base part, a gas input, a gas/liquid output, a reservoir for containing liquid, and a jet for mixing the gas and liquid, the jet being mounted within the base part by means of a generally conical wall ending from the jet to an outer wall of the base part.

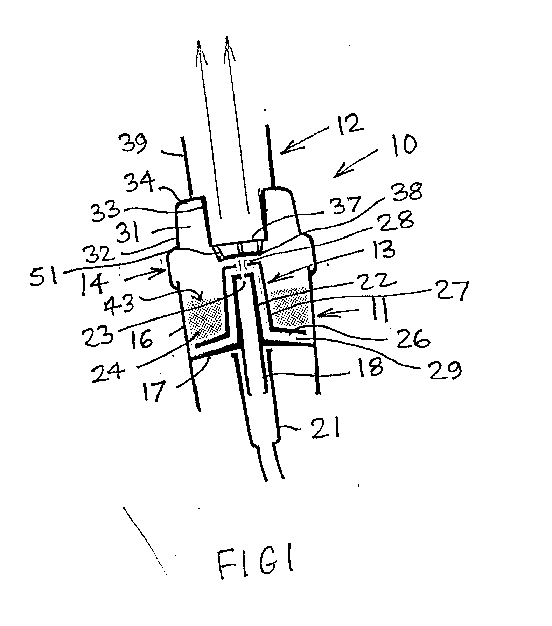

In prior arrangements (seen as that shown in Figure 1) the jet is normally mounted on a part (usually tubular) which extends axially away from the supporting transverse wall forming the base of the reservoir within the nebulizer and this has meant that the jet can move radially owing to flexure of the transverse wall. In the present arrangement, the jet is formed in thc transverse wall itself and, therefore, is unlikely to move from its centralposition even if the wall does flex. The use of a conical wall may allow the nebulizer to be used in different orientations.

In a preferred arrangement of the invention, the jet is provided at the apex of the conical wall.

An input connector for gas may extend axially from the under surface of the transverse wall. It is the provision of this connector attached to the transverse wall that may cause the transverse wall to flex because of relative movement of the nebulizer and incoming gas line.

In order for the nebulizer of the invention to contain sufficient water (because of the provision of a conical wall in place of a substantially flat transverse wall, which reduces the volume of the reservoir for a given diameter) it is necessary for the nebulizer to be somewhat wider than those generally available, but this has the added advantage of making the nebulizer more convenient to fill and also more stable when placed on a surface during the filling operation.

In a further aspect of the invention the nebulizer may comprise a reservoir, a wall of the reservoir itself forming an output connector for the nebulized gas and liquid aerosol In this way the normal cylindrical wall which extends from the reservoir and forms the output connector is replaced by a suitably dimensioned wall of the reservoir itself and thereby the overall length of the nebulizer can be reduced. This makes the nebulizer more convenient to use.

A nebulizer incorporating the various aspects of the invention will now be described by way of example only and with reference to the accompanying drawings in which:

Figure 1 is an axial section of a known nebulizer,

Figure 2 is an axial section of a nebulizer according to the present invention in a vertical orientation,

Figure 3 is an axial section of the nebulizer of Figure 2 in a horizontal orientation,

Figure 4 is a perspective view of the nebulizer of Figure 2, and,

Figures 5, 6 and 7 are side views of the three components forming the nebulizer of Figure 2.

Referring now to Figure 1 there is shown a known nebulizer 10 moulded of rigid plastics material in three separable parts, a base part 11, a cap part 12 and a flange plate 13. The base part 11 is screw threadedly engaged with the cap part 12 by means of a continuous screw thread at 14 (not shown in detail).

The base part 11 comprises a generally cylindrical outer wall 16 a transverse wall 17 extending across the lower part of the cylindrical outer wall 16 with a slight upward slope to its centre so that it comprises a shallow conical wall. Passing through the axial centre of the transverse wall 17 is the gas inlet 18, the gas inlet being defined by a gas inlet connector 19 extending downwardly from the transverse wall 17 to which a standard type gas supply tube 21 may be connected. The gas inlet 18 also extends upwardly from the transverse wall 17 by means of the extending tube portion 22, the upper end of the tube portion 22 being generally in the plane of the upper end of the cylindrical outer wall 16 and being closed by an end wall which includes an aperture therein forming a gas jet 23. It will be understood that the volume between the inner surface of the cylindrical outer wall 16 and the outer surface of the tube portion 22 forms a reservoir 24 for liquid.

The flange plate 13 comprises a shallow conical skirt portion 26 and an upright tubular part 27 extending from the skirt 26, the upper end of the tubular part 27 being closed, but including a gas/liquid mixing jet 28.

In use the configuration is such that a very narrow gap (forming a capillary path 29) is formed between the undersurface oi the skirt part 26 and the upper surface of the transverse wall 17 and between the inner wall of the tubular part 27 and the outer wall of the tube portion 22 whereby liquid in the reservoir 24 may be drawn up through this capillary path 29 from the circular outer lip of the skirt part 26 within the rescrvoir up to the gas/liquid jet 28.

The cap part 12 comprises a screw threaded portion to engage with the base part 11, and a substantially closed cylindrical upper portion which forms a reservoir 31 for the liquid within the reservoir 24 when the nebulizer 10 is disposed horizontally. The reservoir 31 is provided between the outer cylindrical wall 32 and an inner cylindrical wall 33, the two cylindrical walls 32 33 being joined at their outer end by a generally transverse annular wall 34, an upstanding cylindrical portion extending from this annular wall 34 to provide an outlet connector for the gas/liquid mixture. A transverse wall 37 closes the inner end of the inner cylindrical wall 33 and mounts a baffle plate 38 which is disposed just above the gas/liquid mixing jet 28. The undersurface of the baffle plate 38 includes a shaped semi-cylindrical baffle 41 aligned with the gas/liquid mixing jet 28.Aperture means (not shown) is provided to allow the nebulized gas/liquid mixture to pass from within the nebulizer to the output connector 39, which may be provided by apertures in the diaphram wall 37.

In operation, the cap part 12 is removed from the base part 11 and the necessary liquid to be nebulized is poured into the reservoir 24. The cap part 12 is then re-engaged with the base part 11 and the gas supply tube 21 is connected to the gas inlet connector 19. Gas may then be passed under pressure through to the gas nozzle 23 and the action of this causes liquid to be drawn up through the capillary path 29 and to be mixed with the gas and nebulized at the gas/liquid mixing jet 28. By careful design of the jet sizes and relationship with the baffle plate 38 a nebulized mixture of gas and liquid is ejected from the region of the gas/liquid mixing jet 28 and passes out to the output connector 39.

Although such a nebulizer works well, there have been a number of problems as set out in the introduction above. Thus, relative movement of the tube 21 and the nebulizer will cause the transverse wall 17 to flex very slightly and this causes movement of the gas/liquid mixing jet 28 with respect to the baffle 41. This movement can affect the nebulization effect.

Furthermore, the axial length of the nebulizer of Figure 1 is considerable and it would be desireable to make a more compact arrangement.

It is difficult for old people to unscrew the cap part 12 and to fill the reservoir 24, because of its relatively small size and this is particularly important, because any spillage will affect the amount of medication being applied to the patient.

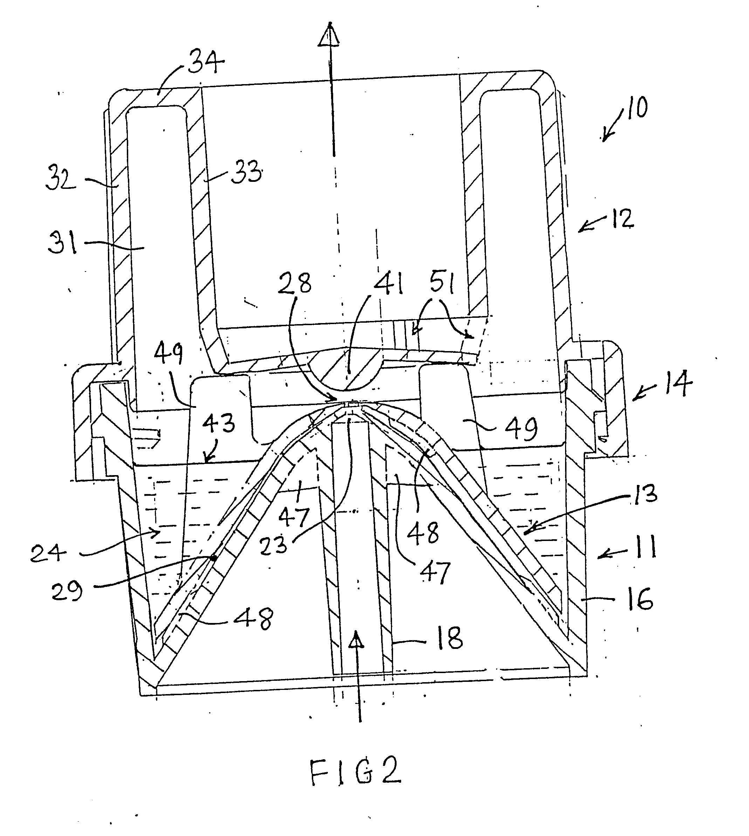

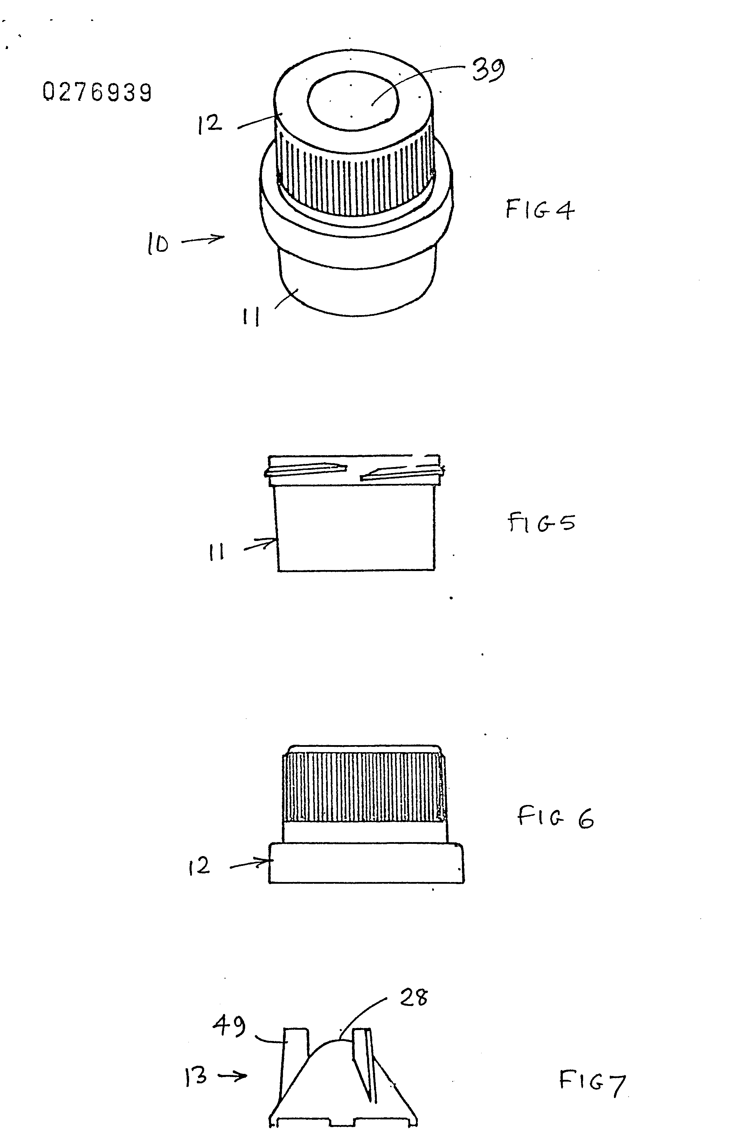

A nebulizer according to the invention is illustrated in Figures 2-7. Similar parts are indicated with the same reference numerals and the general arrangement will not be described further, as it is generally similar to the nebulizer of Figure 1.

In general terms it will be seen that the nebulizer of the invention is considerably shorter in axial length than the nebulizer of Figure 1 thereby making it more compact, the reservoir 31 is somewhat wider which makes it easier to fill. Other advantages will become apparent.

In the base part 11 of the nebulizer according to the invention the generally transverse wall 17 and tube portion 22 is replaced by a wall 46 which is more steeply conical than the transverse wall 17 of Figure 1. Thus, the outer periphery of the closure wall 46 is disposed generally adjacent to the base of the cylindrical wall 16 and extends upwardly at an acute angle between 20° and 60°,preferably 45°, although with a curved central part. The gas inlet connector 19 is mounted directly in this closure wall 46 on the axis of the nebulizer. The gas inlet connector 19 extends downwardly from this central region of the closure wall 46 to a point just above the lower edge of the nebulizer. There are also provided strengthening flanges 47 which extend between the upper end of the gas inlet connector 19 and the under side of the closure wall 46 adjacent the central area.

Similarly, the flange plate 13 is shaped so as to follow the contours of the upper surface of the closure wall 46 and thereby comprises a generally conical shape, the shape of the under surface of the flange plate 13 closely matching the upper surface of the closure wall 46 to provide therebetween capillary path 29. Spacing between the cap part 12 and closure wall 46 is maintained by spacers moulded to the top surface of the closure wall 46 and the under surface of flange plate 19. The gas/liquid mixing jet 28 comprises a jet passing through the axis of the flange plate 13.

The spray nozzle part also includes upwardly extending iingers 49 (3 in number) which may be more readily gripped to allow removal of the flange plate 13 from the base part 11.

Because the shape of the reservoir 24 has been changed by means of the conical lower wall of the reservoir, to maintain the same volume, the outer diameter of the cylindrical outer wall 16 must be increased from 35mm to 42mm.

This increase in diameter also allows changes in the cap part 12. As will be understood from Figure 1, in order to connect with standard tubing the output connector 39 has specific dimensions and in particular, has an inner diameter of 22 mm with a specific taper. Because of the increase in diameter of the nebulizer it is now possible to arrange for the inner cylindrical wall 33 to be of 22 mm diameter and this means that this inner cylindrical wall 33 can itself form the output nozzle 39.

The baffle plate 38 and baffle 41are combined with the wall 37, the baffle 41 comprising a divergent surface diverging away from the jet 28. The baffle 41 in the prefered arrangement comprises a semi spherical protrusion from the underside of the wall 37. Thus the distance between the baffle 41 and the top surface of the flange plate 13 rapidly diverged from one another within increasing distance from the jet. It has been found that the particular arrangement of surfaces creates a very good nebulizer.

Apertures 51 are provided around the inner end of the inner cylindrical wall 33 to allow the nebulized gas liquid mixture to pass out of the interior of the nebulizer to the output connector 39. To prevent the cylindrical connecter portion of the tube engaging with the output connector 39 from covering the apertures 51, the apertures 51 are provided in a inwardly extending conical inner part 52 of the cylindrical wall 33.

Instead of continuous threads being provided on the threaded portions of the base part 11 and cap part 12 there are provided interrupted thread portions which require less turning to tighten than a complete screw thread.

It will be undertood that all parts of the nebulizer are moulded from the same rigid plastic which may be rigid polystyrene (styrene butadiene block copolymer).

Figure 2 shows the nebulizer according to the invention in the vertical orientation and the level of liquid within the reservoir 24 is illustrated at 43. It cam be seen that this liquid level is below the gas/liquid mixing jet 28.

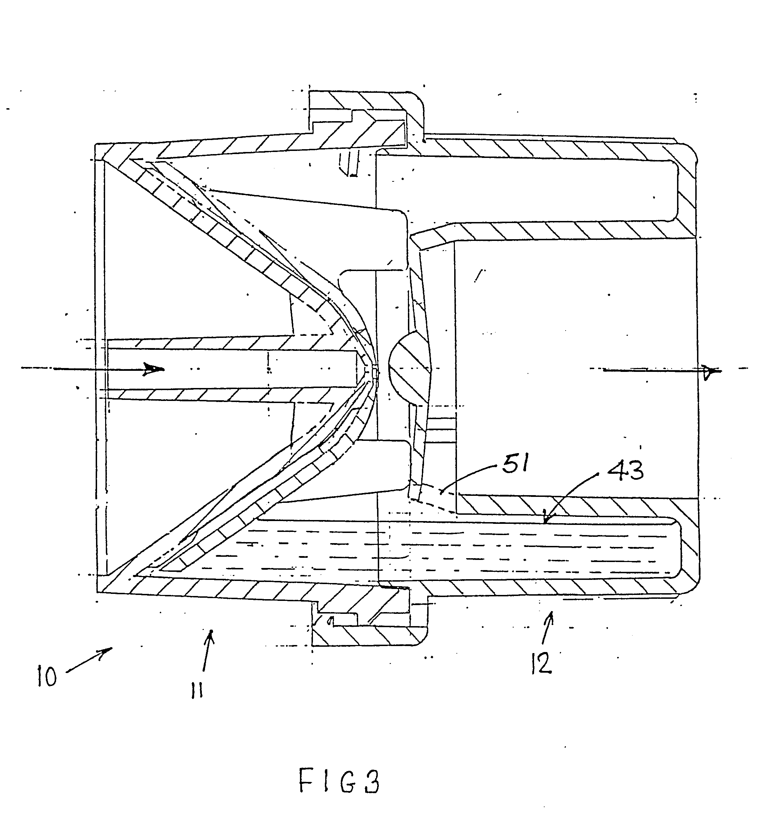

Figure 3 shows the nebulizer when in the horizontal orientation from which it will be seen that the level of the liquid 43 (which is now distributed between the reservoir 24 and the reservoir 31 is still below the gas/liquid mixing jet 28 and the apertures 51 so that no spillage occurs. Indeed, even if the nebulizer were to be inverted completely then all of the liquid would be retained within the reservoir 43 without any spillage out of the apertures 51.

From Figure 3 it would be seen in particular that even in the horizontal position liquid will be drawn up through the capillary 29 as the flange plate 13 extends down to the liquid.

The improvements in this nebulizer will be readily apparent. The nebulizer is more compact so far as its axial length is concerned (50mm compared with 75mm for our earlier nebulizer of Figure 1), because the output connector 39 has been removed and replaced by an enlarged inner cylindrical wall 33. The elimination of the tube portion 22 means that the inner end of the gas supply tube 21 when engaged with the gas inlet connector 19 is now adjacent to the gas/liquid jet 28 and this further reduces the axial length as well as allowing a better form of capillary path 29 which improves the liquid flow therethrough.

To compensate for this, the nebulizer is wider and in particular the base part 11 is of greater diameter which renders it more stable when it is placed on a surface for filling. This enables easier filling of the reservoir by an elderly person and less likelihood of spillage.

The gas/liquid mixing jet 28 and gas nozzle 23 are more rigidly mounted with respect to the outer cylindrical wall 16 as they are directly connected by the conical closure wall 46. Furthermore, the attachment of the gas inlet connector 19 directly to the underside of the gas nozzle 23 means that even if that gas inlet connector 19 moves slightly, it will have very little effect on the position of the gas jet and there will not be leverage of that movement by the upwardly exterlding tube portion 22 of the nebulizer of Figure 1. In any case, the flexure of the tube portion 22 and the closure wall 46 is further resisted by the flanges 47.

It is well known that the three parts of the nebulizer must be separated for regular cleaning and removal of the flange plate 13 has been particularly difficult with the arrangement of the nebulizer oi Figure 1. The increased diameter of the nebulizer and the provision of the fingers 49 simplifies removal of the flange plate 13 since these can be more readily gripped for removal thereof.

A considerably improved nebulizer according to the invention has been described. However,the invention is not restricted to the details of the foregoing example, but many obvious alternatives are incorporated within the scope of the invention.

## Claims

1. A nebulizer comprising a base part, a gas input, a gas/liquid output, a reservoir for containing liquid, and a jet for mixing the gas and liquid, the jet being mounted within the base part by means of a generally conical wall extending from the jet to an outer wall of the base part.

2. A nebulizer as claimed in Claim 1 in which the jet is provided at the apex of the generally conical wall.

3. A nebulizer as claimed in claim 1 in which an input connector for gas extends axially from the under surface of the generally conical wall.

4. A nebulizer as claimed in any of claim 1 in which a wall of the reservoir itself forms the gas/liquid output for the nebulized gas and liquid aerosol.

5. A nebulizer comprising a reservoir, the reservoir having a side wall and a base wall, the base wall being generally conical and extending from a lower outer periphery adjacent the side wall to a generally central raised part, a gas input being provided in the generally central raised part and a jet being mounted adjacent thereto, a capillary path being provided from said lower outer periphery to the jet, the nebulizer further including a gas/liquid output.

6. A nebulizer as claimed in claim 5 in which a cover is provided for said reservoir, said cover incorporating a gas/liquid outlet.

7. anebulizer as claimed in claim 5 or claim 6 in which said reservoir includes a cover, said cover extending adjacent said jet and including, opposite said jet, a divergent baffle part.

8. A nebulizer as claimed in any of claims 5 to 7 in which said capillary path is formed by a flange plate extending from adjacent said lower outer periphery of the base wall to the jet, said flange plate being spaced from said base wall by a sufficiently small distance to maintain capillary action in said space, said jet being mounted in said flange plate.

9. A nebulizer as claimed in claims 7 and 8 in which the surface of said flange plate facing said cover is divergent adjacent said jet.

0276939

FIG 1

0276939

FIG 2

0276939

FIG3

0276939

FIG 4

FIG 5

FIG 6

FIG 7